# FASCICULE DE BREVET EUROPEEN

(11) **EP 1 219 947 B1**
(45) Date de publication et mention de la délivrance du brevet: **18.10.2006**
(21) Numéro de dépôt: 00811215.3
(22) Date de dépôt: 20.12.2000
(51) Int. Cl.: G01N 11/10, G01N 33/15, G01N 33/04, G01N 3/08

(54) **Procédé de mesure pénétrométrique de la consistance de substances et dispositif de mesure pour la mise en oeuvre du procédé**
Verfahren zur penetrometrischen Messung der Konsistenz von Substanzen und Vorrichtung zur Umsetzung des Verfahrens
Method for penetrometrically measuring the consistency of substances and apparatus for the application of the method

(43) Date de publication de la demande: 03.07.2002
(73) Titulaire: Consismetric S.A., 1510 Moudon (CH)
(72) Inventeur: Aubort, Pierre, 1510 Moudon (CH)
(74) Mandataire: Ganguillet, Cyril

(56) Documents cités:
- EP-A- 0 439 405
- EP-A- 0 542 188
- DE-A- 2 929 137
- DE-A- 2 932 288
- FR-A- 2 491 213
- FR-A- 2 595 824
- RO-B- 107 317
- US-A- 4 611 928
- US-A- 5 496 973
- WINTHER G ET AL: "A UNIVERSAL AXIAL RHEOMETER FOR THE CHARACTERIZATION OF VISCOELASTIC MATERIALS" APPLIED POLYMER SYMPOSIA,US,JOHN WILEY AND SONS,INC. NEW YORK, vol. 48, 23 juillet 1990 (1990-07-23), pages 493-502, XP000228052 ISSN: 0570-4898
- PATENT ABSTRACTS OF JAPAN vol. 1995, no. 08, 29 septembre 1995 (1995-09-29) & JP 07 120371 A (SHIGETA SEISAKUSHO:KK), 12 mai 1995 (1995-05-12)
- LAWLOR M.S. ET AL.: "Correlation of mechanical/textural properties of pharmaceutical gels to texture analyser instrumental parameters" JOURNAL OF PHARMACY AND PHARMACOLOGY, 136TH BRITISH PHARMACEUTICAL CONFERENCE SCIENCE PROCEEDINGS, vol. 51, septembre 1999 (1999-09), page 239 XP000995596

## Description

La présente invention concerne un procédé de mesure pénétrométrique de la consistance d'une substance, dans lequel on place un échantillon de ladite substance sur un support mobile constituant un plateau pour l'échantillon, un capteur de force supportant ledit plateau, on fait pénétrer du haut vers le bas une sonde calibrée dans cet échantillon, selon une course à accomplir dans la masse de l'échantillon et selon une vitesse préalablement choisie, et on mesure la force verticale dirigée vers le bas appliquée audit support à au moins un moment prédéterminé de la course de la sonde. L'invention concerne également un dispositif de mesure pour la mise en oeuvre du procédé.

Le besoin de connaître les propriétés viscoélastiques de substances ou mélanges s'exprime dans des domaines divers, par exemple les industries chimiques, pharmaceutique, cosmétique, alimentaire, pétrochimique et métallurgique. De nombreux procédés de mesure et leurs dispositifs de mise en oeuvre ont été proposés.

On peut mentionner les techniques mesurant les propriétés d'écoulement de l'échantillon testé. Ces techniques s'appliquent à des liquides newtoniens, comme le lait par exemple. Lorsqu'il s'agit de mesurer les caractéristiques de viscoélasticité de substances ou mélanges non newtoniens, tels que pâtes et pommades, la mesure de l'écoulement ne peut s'appliquer car l'écoulement de tels échantillons est nul ou non raisonnablement mesurable. Il en va de même de mélanges thixotropiques donnés par certains types de yoghourts.

Faute de pouvoir mesurer utilement un écoulement, on s'est appliqué à mesurer les paramètres de cisaillement du matériau dans les rhéomètres ou viscosimètres à mobile tournant. L'échantillon est placé dans un godet formant le stator, dans lequel on engage un rotor qui peut avoir diverses formes selon le type d'appareil. Le rotor est mis en mouvement grâce à un moteur; plus la viscosité est importante, plus le rotor est freiné. On lit sur une échelle de viscosités la valeur d'une résistance provoquée par la viscosité du matériau situé dans l'entrefer entre rotor et stator.

Ce type de procédé et de dispositif donnent satisfaction lorsqu'il s'agit d'apprécier comparativement au même moment la viscosité de mélanges différents. Ainsi, si l'on réalise plusieurs échantillons d'une même crème en variant par exemple les proportions de l'un des ingrédients, ces systèmes permettent de qualifier et ordonner les échantillons du moins visqueux au plus visqueux. En revanche, ces systèmes ne permettent pas de comparer valablement dans le temps les propriétés de consistance d'une seule et même crème à divers degrés de vieillissement ou de maturité, et telle qu'elle se présente dans son emballage. En effet, un grave défaut des systèmes rotor-stator pour la mesure de l'évolution de la viscosité dans le temps est qu'ils nécessitent avant chaque mesure une manipulation de l'échantillon pour son introduction dans le stator. Or, cette manipulation influe sur la consistance de l'échantillon. En d'autres termes, on modifie avant de les mesurer les paramètres que l'on cherche justement à déterminer avec la plus grande précision possible.

Ces constatations ont conduit à tenter d'exprimer la consistance de pâtes et crèmes par la mesure de paramètres de pénétration d'une sonde calibrée dans un échantillon. Les dispositifs connus, adaptés à cette méthode de mesure, appelée consistométrie ou pénétrométrie, ont comme principe commun de faire pénétrer une sonde d'un calibre et/ou d'un poids déterminé, par exemple un cylindre ou un cône renversé, dans la masse de l'échantillon, mais expriment sa consistance selon diverses grandeurs mesurées, de sorte qu'en général, ils sont non compatibles entre eux. Ces systèmes peuvent se fonder sur la profondeur de pénétration atteinte en un temps donné, - c'est par exemple le cas de la méthode préconisée par la pharmacopée helvétique -, ou sur le temps mis par la sonde pour effectuer un certain trajet, ou encore sur la force à exercer pour que la sonde accomplisse un certain trajet en un certain temps, à une température déterminée, et ont donné lieu à l'élaboration de nombreux protocoles de mesure, d'appareils et de sondes destinés à les mettre en oeuvre.

Dans certains dispositifs connus, basés sur la mesure d'une force à exercer, la résistance offerte par l'échantillon à la pénétration de la sonde est perçue par un ou plusieurs dynamomètres solidaires de la sonde et traduite sur une échelle métrique pour en permettre la lecture. Ces appareils présentent cependant des inconvénients. En premier lieu, ces appareils sont directement dépendants des qualités et limites du dynamomètre utilisé. Pour pallier cette dépendance, on a proposé des systèmes dans lesquels la mesure de pénétration est fournie par une lecture de la résultante du déplacement d'un jeu de plusieurs dynamomètres. Ce système a cependant le désavantage de rendre la mesure dépendante non seulement de la qualité et de la stabilité des dynamomètres, mais également de celles de leur assemblage. De plus, il est particulièrement difficile de déceler une anomalie dans un dynamomètre en réseau, ce qui ne fait qu'ajouter à la difficulté qu'il y a à diagnostiquer un éventuel dérèglement d'un dynamomètre, puis à y remédier.

Dans les pénétromètres commercialisés par exemple par les sociétés Sommer et Runge KG (RFA), Stevens Ltd (GB) ou Lloyd Instruments Ltd (GB), qui mettent en oeuvre un procédé du type défini d'entrée, un moteur fait descendre une sonde, fixée à un support mobile en translation verticale, dans l'échantillon, qui est placé sur un plateau fixe. Un capteur de force solidaire de la sonde et/ou de son support mobile mesure la réaction de l'échantillon à la pénétration de la sonde, c'est-à-dire une force de réaction dirigée vers le haut. La résultante des forces agissant sur le capteur comprend une ou plusieurs des composantes suivantes : le poids de la sonde, une partie du poids du support, un couple de torsion dû au bras de levier entre le point d'application de la sonde et l'axe de translation du support mobile ou le point de fixation du capteur; les composantes précédentes doivent être soustraites du signal total mesuré, par exemple par une mise à zéro. Le signal restant comprend la poussée d'Archimède de l'échantillon sur la sonde, et enfin la force de réaction viscoélastique proprement dite, due à la consistance et à la nature de l'échantillon. Ces appareils sont apparemment faciles d'emploi, mais ne sont pas exempts de défauts. Un très léger jeu entre pièces mobiles et pièces fixes assurant le mouvement de descente de la sonde peut modifier le couple de torsion précité et introduire ou modifier une composante de frottement, sans que cette variation parasite de la force totale captée par le capteur ne soit détectée en tant que telle pendant la mesure. L'étalonnage périodique de ces appareils se fait en suspendant des poids calibrés au support mobile, c'est-à-dire au moyen d'une force de traction sur le capteur de force, alors que pendant une mesure pénétrométrique, la force à mesurer est une force de compression, de sorte que cet étalonnage est effectué en dehors de la plage de travail.

Le document FR-2 595 824 décrit un dispositif de mesure pénétrométrique avec les caractéristiques figurant dans le préambule de la revendication 5. Un micro-ordinateur commande le déplacement en translation d'un embout, par l'intermédiaire d'un moteur, en fonction d'une mesure de force donnée par une balance.

La présente invention a pour but de proposer un procédé de mesure de la consistance par pénétrométrie, ainsi qu'un dispositif pour la mise en oeuvre du procédé, destinés à éliminer les éléments d'incertitude des dispositifs de l'art antérieur et offrant une grande facilité et sûreté d'étalonnage.

Ces buts sont atteints par un procédé dont les caractéristiques sont énoncées à la revendication 1; des modes d'exécution de ce procédé font l'objet des revendications 2 à 4. Les caractéristiques du dispositif selon l'invention sont énoncées à la revendication 5; les caractéristiques de modes d'exécution du dispositif sont plus particulièrement énoncées dans les revendications 6 à 15.

Dans le procédé selon l'invention, la mesure de la consistance est exprimée par la mesure d'une force dirigée vers le bas, exactement opposée à la réaction de l'échantillon à la pénétration de la sonde, après soustraction du poids propre de l'échantillon, y compris son récipient s'il y lieu, et du support. Il est très facile de déterminer si l'appareil donne une mesure exacte et d'étalonner celui-ci en simulant la force ci-dessus par des poids calibrés disposés sur le support. Dans le procédé selon l'invention, le poids propre de la sonde n'a aucune influence, contrairement au procédé utilisé par les dispositifs de l'art antérieur dans lesquels la sonde et le capteur de force sont directement reliés. De même, les frottements affectant les pièces du dispositif assurant la montée et la descente de la sonde n'ont aucune influence sur le résultat de la mesure.

On donne ci-après une description détaillée de modes d'exécution du procédé et du dispositif selon l'invention en se référant au dessin où:
- la figure 1 représente, de façon schématique, un premier mode d'exécution du dispositif selon l'invention;
- la figure 2 représente un autre mode d'exécution du dispositif selon l'invention dans une vue en perspective;
- la figure 3 représente le mode d'exécution de la figure 2, le carénage de l'appareil de mesure étant enlevé;
- la figure 4 illustre un enregistrement graphique d'une séquence de mesure pratiquée sur un premier produit au moyen du dispositif illustré à la figure 1;
- la figure 5 illustre un enregistrement graphique d'une séquence de mesure pratiquée sur un deuxième produit au moyen du dispositif illustré à la figure 1;
- la figure 6 illustre une série de mesures effectuées sur un même produit à divers degrés de vieillissement, au moyen du dispositif illustré à la figure 1 ; et
- la figure 7 illustre une série de mesures effectuées sur un même produit semi-solide à divers degrés de vieillissement, au moyen du dispositif illustré à la figure 2.
- la figure 8 illustre une série de mesures effectuées sur un même produit semi-solide à divers degrés de vieillissement, au moyen du dispositif illustré à la figure 2.

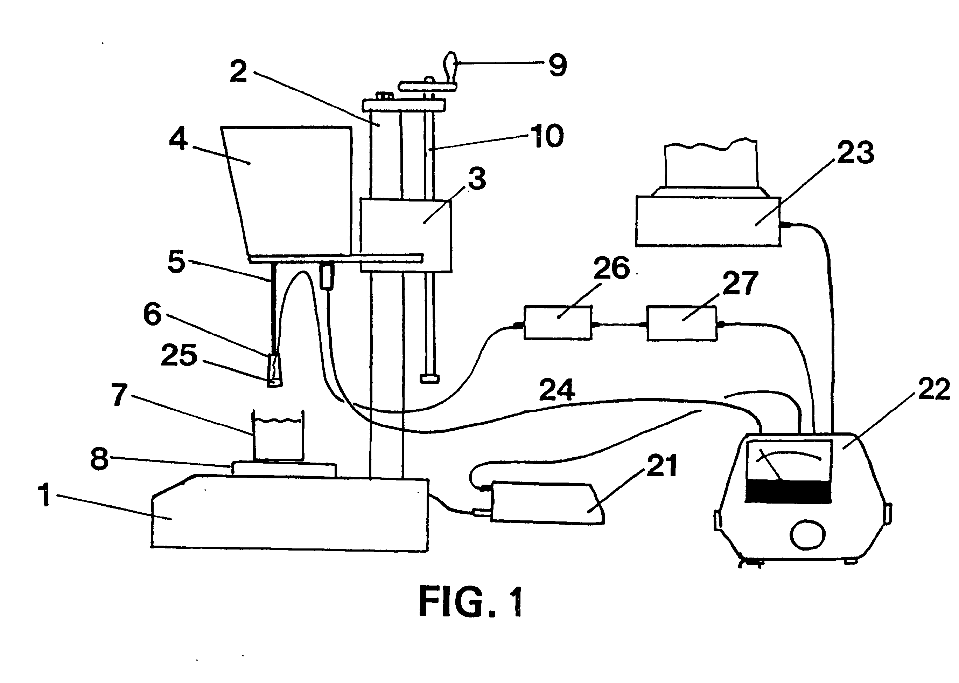

Sur la figure 1, on reconnaît un socle avec un capot 1 qui donne son assise à l'ensemble du système de mesure et une colonne support 2 sur laquelle peut coulisser une bague de réglage et de fixation 3. La bague 3 est engagée sur et en prise avec une tige filetée 10 tournante. Elle peut être déplacée manuellement et bloquée à la hauteur désirée sur la colonne 2 grâce à la tige filetée 10 et à une manivelle 9. Cette bague de fixation sert de support à un coffret moteur 4 qui comporte un moteur pas à pas à broche filetée. Ce moteur permet de faire monter et descendre une tige de poussée 5 sur laquelle est fixée une sonde 6 qui pénétrera dans la substance dont la consistance doit être mesurée. Des moteurs de divers types peuvent être utilisés, pourvu qu'un comptage d'impulsions soit possible, que celui-ci ait lieu de toute façon (moteur à courant alternatif, moteur pas à pas, moteur linéaire) ou qu'il soit le résultat d'un générateur d'impulsions spécialement dédié (moteur à courant continu avec codeur). Un moteur du type Sonceboz 7220 R010, qui a une course maximum de 63.5 mm, une force maximale de 15.3 N, un pas linéaire de 0.0508 mm et qui fonctionne sous 12 volts convient parfaitement pour une application destinée au test de substances semi-fluides à semi-dures, par exemple de crèmes cosmétiques. Le mouvement qui est imprimé par le moteur à la tige 5 fait l'objet de présélections qui déterminent la course. Dans ce mode de réalisation, cinq courses prédéterminées sont choisies à 10, 20, 30, 40 et 50 mm. La vitesse de descente de la tige 5 fait aussi l'objet de valeurs prédéterminées : On a retenu des valeurs de 0.05 - 0.1 - 0.25 - 0.5 et 2.5 millimètres par seconde. Les indications précédentes concernant les présélections sont données à titre d'exemple et peuvent évidemment être modifiées en fonction des applications.

Le capot 1 contient un capteur de force (non-visible sur la figure 1) surmonté d'un plateau 8, mesurant un poids ou toute autre force verticale dirigée vers le bas s'exerçant sur le plateau 8, qui est agencé de façon à ne pas toucher le capot 1. On utilise de préférence un capteur, par exemple à quartz piézoélectrique, dont le signal de sortie est digitalisé. On reconnaît finalement sur la figure 1 un échantillon 7, ici un récipient, avec la substance à tester, posé sur le plateau 8. Comme le montre la figure 1, l'échantillon peut être testé dans des conteneurs ou emballages d'origine de dimensions variées, sans manipulation préalable de la substance.

Un dispositif de commande électronique, auquel l'expérimentateur entre les paramètres opératoires sélectionnés au moyen de boutons appropriés, est logé dans le coffret moteur 4. Le dispositif de commande comporte un codeur ou compteur d'impulsions délivrées au moteur par ce dispositif de commande, pour déterminer la course de la sonde. Ce dispositif de commande comporte également une horloge interne délivrant un signal de fréquence, et détermine de la sorte la fréquence des impulsions délivrées au moteur et donc la vitesse de déplacement de la sonde. Le capteur de force est également couplé au dispositif électronique de commande, qui peut utiliser son signal et l'afficher. Ce dispositif de commande peut délivrer une première commande de mise à zéro du signal du capteur lorsque l'échantillon est placé sur le plateau, avant que la sonde ne soit en contact avec l'échantillon, et une deuxième commande de mise à zéro au moment précis où le contact entre la sonde et l'échantillon est révélé par la première valeur non nulle du signal après la première mise à zéro. Simultanément le dispositif de commande peut fournir à l'organe de comptage d'impulsions une commande de mise à zéro, de sorte que le comptage des impulsions qui détermine la course de la sonde commence exactement à ce moment.

Sur la figure 1, on retrouve un convertisseur digital-analogique 21, relié au capteur et à un bloc analogique 22. Le bloc analogique 22 est relié d'autre part à un enregistreur graphique 23 qui permet le traçage de la courbe des valeurs du signal délivré par le capteur tout au long de la descente de la sonde dans la matière à tester.

Le câble de connexion 24 permet d'une part d'alimenter le coffret-moteur 4 et d'autre part de transmettre le signal représentatif de la descente de la sonde au bloc analogique et à l'enregistreur.

Enfin, La figure 1 montre également un système de mesure de la température, qui est ici composé d'une thermistance 25 incorporée à la sonde, mais qui peut aussi être indépendant de la sonde. La thermistance est reliée à un circuit d'amplification analogique 26, lui-même relié à un organe d'affichage 27 qui peut être numérique ou analogique. Il est ainsi possible de traduire également la mesure de la température, sous forme graphique, en combinaison avec la représentation des autres grandeurs mesurées.

Dans la configuration du dispositif présenté à la figure 1, le procédé de mesure peut se dérouler de la façon suivante : On commence par placer le récipient 7 avec son contenu sur le plateau 8. On choisit une sonde calibrée 6. La sonde peut avoir diverses formes, notamment celle d'un cylindre; elle peut aussi se présenter sous forme d'une tige terminée par une assiette à face plane dont la surface est choisie selon l'application. On fixe la sonde sur la tige de poussée 5 et, avec la manivelle 9, on effectue un positionnement initial rapide de la sonde 6 au-dessus du conteneur 7 en descendant manuellement le coffret moteur jusqu'à ce que la face inférieure de la sonde 6 affleure la surface de la matière à mesurer contenue dans le récipient 7. On effectue 1a mise à zéro du signal délivré par le capteur de force.

On sélectionne la longueur de la course parmi les valeurs prédéterminées; on sélectionne également la vitesse de déplacement de la sonde parmi les valeurs prédéterminées. On commande la mise en marche du moteur qui fait descendre la sonde à la vitesse choisie jusqu'à ce que la course choisie soit atteinte. A ce moment précis, le capteur indique une certaine valeur, laquelle est retenue comme la valeur de la consistance.

Il est préférable de saisir cette valeur par un dispositif électronique qui permet à la fois l'affichage, la mémorisation et la conservation des valeurs mesurées et permet leur traitement informatique. Le dispositif reçoit un ordre de mémorisation de la valeur de la température lorsque la course prédéterminée de la sonde est atteinte, c'est à dire à la fin de la mesure consistométrique.

A condition d'utiliser les mêmes paramètres prédéterminés en ce qui concerne la course et la vitesse de la sonde, on peut soumettre des échantillons d'un même produit à des séances de mesures pratiquées à diverses étapes de vieillissement de ce produit, et déterminer par exemple sa période optimale de commercialisation. La constance de la vitesse de pénétration et de la course de la sonde permettent d'obtenir des valeurs comparables dans le temps, cela d'autant que la précision et l'étalonnage du capteur en sont aussi les garants.

On a indiqué que, lors du début de la mesure, on amène manuellement la surface inférieure de la sonde en contact de la surface de la substance à tester. Cette opération peut être rendue automatique, par le dispositif de commande électronique. On effectue un pré-positionnement rapide de la sonde à faible distance de l'échantillon. Puis le dispositif électronique commande la descente de la sonde et met le signal du capteur à zéro. Lorsque la valeur donnée par le capteur de force devient différente de zéro, de par le contact de la sonde avec l'échantillon à mesurer, un comparateur interne permet d'une part l'envoi d'un signal de mise à zéro du compteur d'impulsions du moteur et d'une commande d'arrêt momentané du moteur, et d'autre part la remise à zéro automatique du signal du capteur de force. Le moteur est ensuite ré-alimenté, et la suite de la mesure est commandée comme déjà décrit.

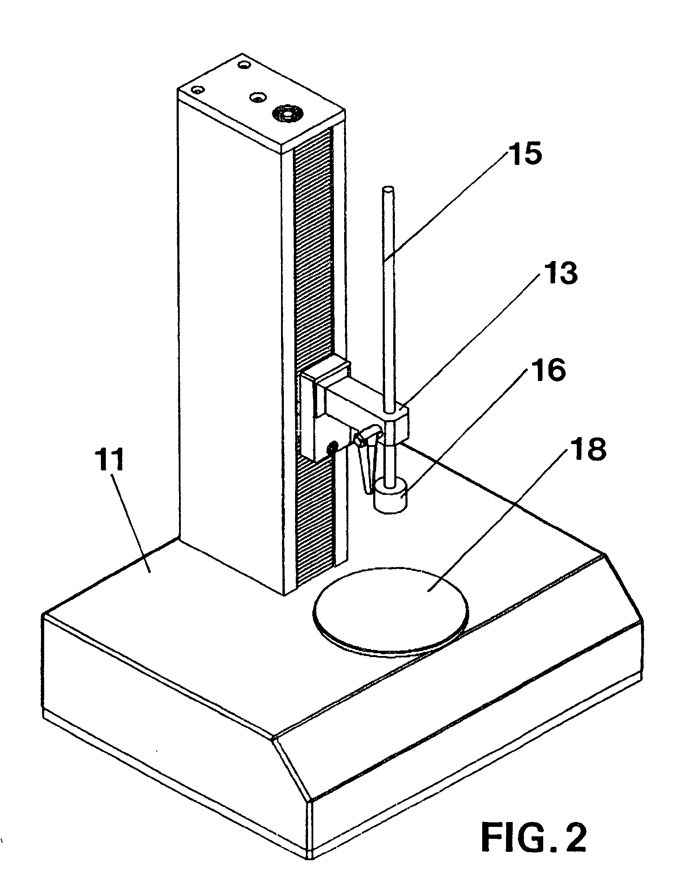

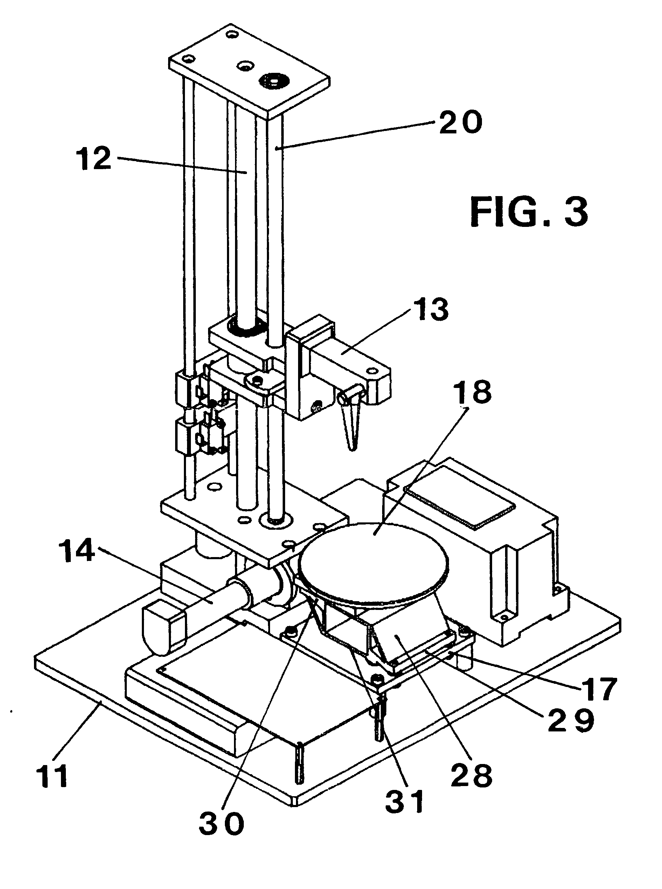

On a ci-dessus décrit un mode d'exécution du dispositif sous forme d'une combinaison de divers éléments 1, 4, 21, 22, 26, 27 reliés entre eux, mais séparés. Le dispositif selon l'invention se présente de préférence comme un appareil intégré comportant ces éléments ou des éléments équivalents dans un même carénage 19. La figure 2 représente cet appareil intégré, muni de son carénage, d'une tige de poussée 15 et d'une sonde 16 montés sur un support de fixation et de réglage 13. La figure 3 représente le même appareil, sans son carénage et sans la tige de poussée. On reconnaît une colonne de guidage 12, le support de fixation et de réglage 13, qui peut coulisser librement sur la colonne 12, ainsi qu'une tige filetée 20, dont la rotation fait monter ou descendre le support 13. La tige filetée 20 est mise en rotation par un moteur 14 installé cette fois dans le bas de l'appareil, et qui entraîne la tige 20 en rotation par l'intermédiaire d'un engrenage. L'ensemble des composantes de l'appareil est monté sur un socle 11. Pour que le plateau 18 porte-échantillon dépasse de la surface supérieure du carénage 19, le capteur de force 28 est monté sur un socle surélevé 17.

Dans ce mode d'exécution, le capteur de force 28 est une cellule de charge à guidage parallèle élastique, commercialisée par la société Digi Sens (CH-Murten). Comme le montre la figure 3, le dispositif de guidage parallèle comprend une portion de fixation 29 au socle 17 et une portion porte-plateau 30, reliées par un parallélogramme 31. Ce dispositif de guidage peut être réalisé sous forme d'un profilé extrudé et tronçonné. Dans le parallélogramme 31 est logé un enregistreur de charge (non-visible sur la figure 3) sollicité directement par la portion porte-plateau 30 et par la portion de fixation 29. Le plateau 18 est solidaire de la portion porte-plateau 30. Le plateau 18 est donc mobile, sa mobilité étant directement liée à la déformabilité de la cellule de charge 28. La flexion du parallélogramme 31 sous l'effet d'une force verticale s'exerçant sur le plateau 18 entraîne un abaissement minime de ce plateau 18, d'une fraction de millimètre sous l'effet d'une force de l'ordre de la centaine de Newton, de sorte que l'erreur résultante sur la course de la sonde dans l'échantillon est négligeable.

L'enregistreur de charge est ici une corde vibrante, et le signal de sortie est sa fréquence de vibration. Ce signal de sortie est digitalisé. A la place d'une corde vibrante, on pourrait également utiliser un quartz piézoélectrique ou un transducteur capacitif. Un dispositif électronique d'acquisition et de traitement de signal et de commande du moteur, basé sur un microprocesseur, peut également être intégré à l'appareil.

Toutefois, pour abaisser le coût de l'appareil, un certain nombre de fonctions peuvent être externalisées et confiées à un micro-ordinateur sur lequel sera implanté un logiciel spécifique, auquel l'appareil illustré par les figures 2 et 3 peut être relié. La connexion de l'appareil de mesure à un micro-ordinateur est assurée soit par l'utilisation d'une liaison RS232 soit par la connexion directe de ports libres de l'ordinateur.

En premier lieu, certaines des fonctions assumées par des composants de l'élément 4 dans le mode d'exécution de la figure 1 sont confiées à l'ordinateur. Il s'agit par exemple d'utiliser l'horloge interne de l'ordinateur au lieu d'en implanter une dans l'appareil. De même, la gestion des paramètres et des commandes et impulsions délivrées au moteur est confié à un logiciel spécifique. Ledit logiciel spécifique gère le comptage des impulsions délivrées au moteur, la délivrance d'un signal de fréquence, la mise à zéro du signal lorsque l'échantillon est placé sur le plateau, ainsi que la mise à zéro du compteur d'impulsions au moment où le contact entre la sonde et l'échantillon est révélé par la première valeur non-nulle du signal, et finalement la collecte des valeurs données par le capteur de force pendant l'enfoncement de la sonde, ou, selon la variante du procédé de mesure choisie, la valeur donnée par le capteur au moment où le compteur d'impulsions atteint un nombre prédéterminé correspondant à la fin de la course de la sonde.

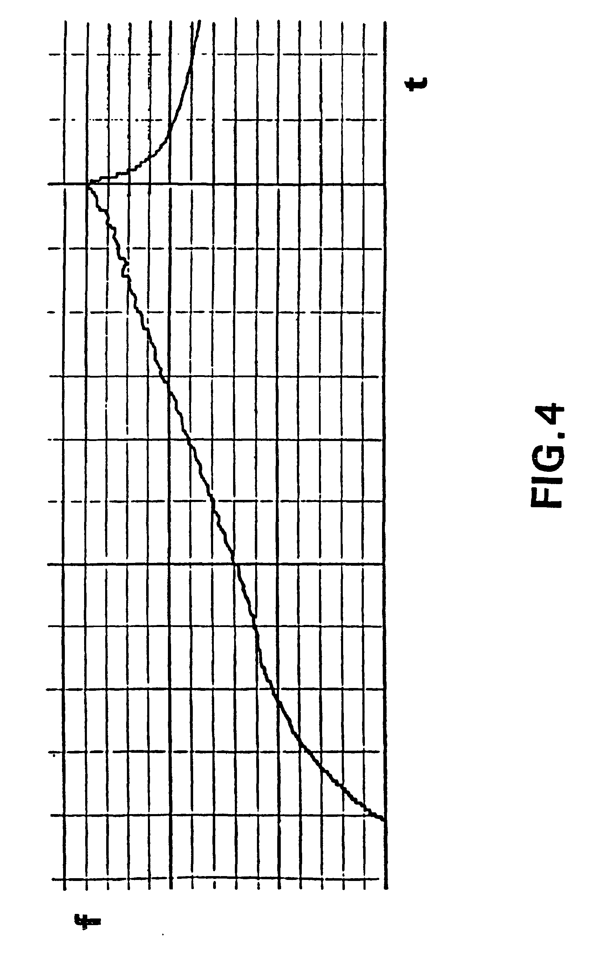

Outre la possibilité de confier à l'ordinateur et à son logiciel spécifique certaines des fonctions nécessaires à la réalisation de la mesure elle-même, l'intérêt de l'interconnexion découle aussi de la possibilité d'utiliser des bases de données et des programmes tels que des tableurs pour stocker les paramètres de mesure de chaque produit testé, en particulier la profondeur de course, la vitesse du moteur et la taille de la sonde, ainsi que les résultats des mesures fournies par le thermomètre. On ajoutera que la présentation de graphiques illustrant les valeurs des mesures et leur comparaison dans le temps, comme ceux faisant l'objet des figures 4 à 7 peut être réalisée grâce aux logiciels et périphériques usuels de l'ordinateur et ne nécessite plus la conversion digital-analogique.

### Exemples d'application

### Exemple 1

La figure 4 est un enregistrement d'une mesure pratiquée sur un échantillon de fromage Tilsit 1/4 gras à l'aide du dispositif de mesure illustré par la figure 1. La température moyenne est de 13°C. La sonde utilisée est une tige d'un diamètre de 2 mm et la profondeur totale de la course est de 20 mm. La vitesse de descente de la sonde est de 0.1 mm/s. La courbe montre la force f, résultante des réactions de l'échantillon à la pénétration de la sonde, en fonction du temps t. La partie croissante de la courbe montre une réaction complexe du fromage à la pénétration. La partie décroissante montre un phénomène de relaxation. La valeur maximale atteinte en bout de course, 140 grammes, est prise comme une valeur empirique de la consistance, qui permet de comparer des échantillons entre eux, par exemple à différents stades de maturation, à condition de conserver des conditions opératoires constantes.

### Exemple 2

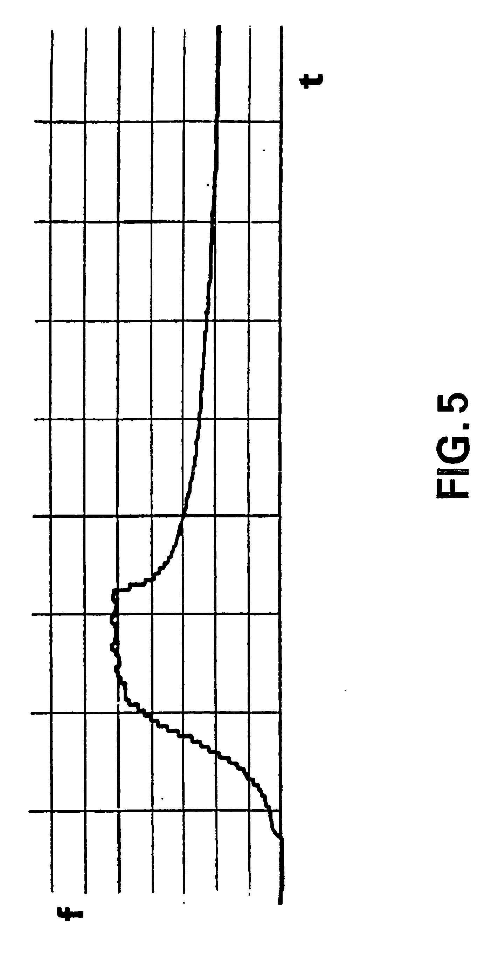

La figure 5 est un enregistrement d'une mesure pratiquée sur un échantillon de crème pharmaceutique à l'aide du même dispositif. La température moyenne est de 13°C. La sonde utilisée est une tige fine terminée par une assiette d'un diamètre de 15 mm et la profondeur de course est de 50 mm. La vitesse de descente de la sonde est de 0.5 mm/s. La force de réaction f enregistrée atteint une valeur maximale de 50 g, avant la fin de la course. Cette valeur est prise comme valeur empirique de la consistance.

### Exemple 3

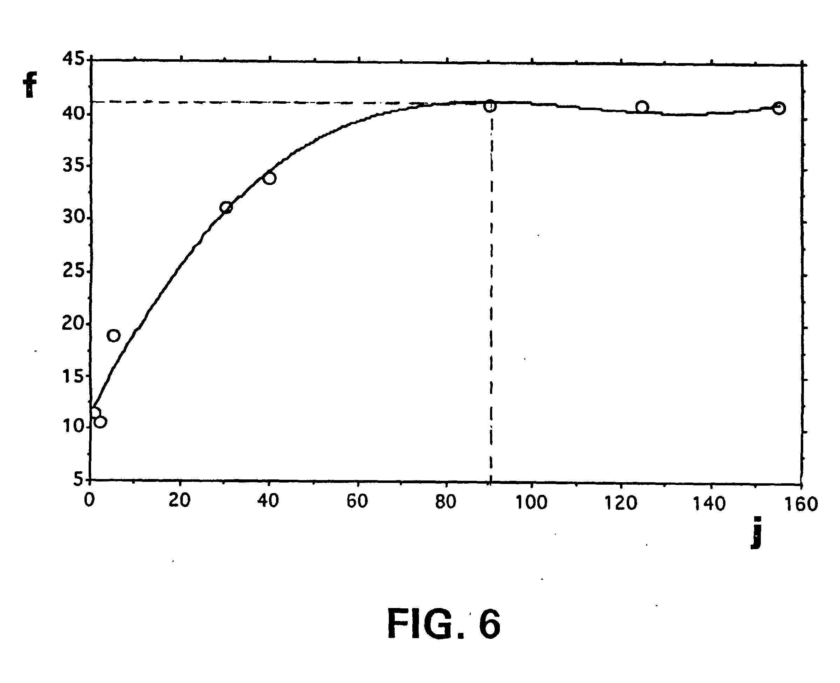

La figure 6 est une courbe reflétant les résultats de plusieurs mesures prises à différentes dates, mais toujours dans les mêmes conditions, sur un échantillon de crème cosmétique. L'abscisse exprime la durée j en jours, à partir de la date de fabrication, et l'ordonnée la mesure f en grammes lue à la fin de la course de la sonde, comme dans l'exemple 2. Toutes les mesures indiquées sont exécutées à une température de 20.5°C. La sonde utilisée a une face de contact plane dont la surface est de 1 cm², la profondeur présélectionnée est de 30 mm et la vitesse de descente de la sonde est de 0.25 mm/s.

La courbe montre que la consistance de la crème, après une augmentation initiale, atteint une valeur à peu près constante au bout de 70 jours. Cette donnée est avantageusement prise en compte dans le cadre de la commercialisation du produit pour prévoir un stockage intermédiaire entre la date de fabrication et la date de commercialisation. En effet, on sait qu'il existe chez le consommateur un phénomène de "psycho-acceptance" des crèmes : pour être bien acceptées, celles-ci doivent avoir une consistance dans une plage optimale qui, pour cet exemple, se situe entre 35 et 55 grammes et ne doit pas varier, ni d'un pot à l'autre d'un même lot ou de lots différents d'un même produit, ni après le début d'utilisation d'un pot donné.

### Exemple 4

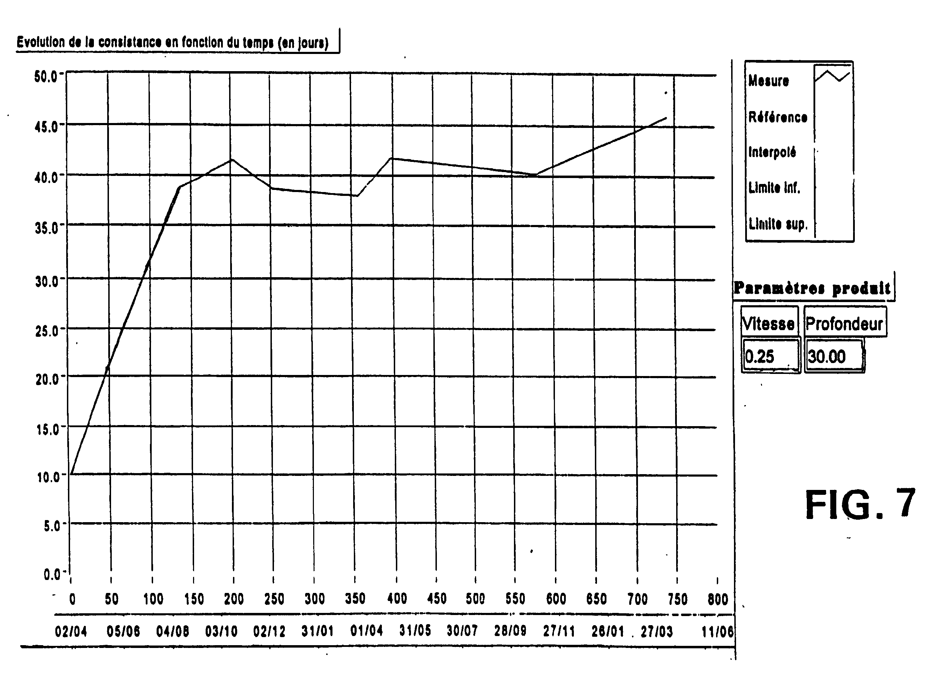

Quatorze lots d'une même formulation de crème semi-solide ont été fabriqués de façon identique, successivement, au cours d'une période de deux années. La mesure de leurs consistances au cours du temps a été effectuée dans les mêmes conditions opératoires, température de 20°C, vitesse de descente de la sonde 0,25 mm/s, course de 30 mm, à l'aide du dispositif informatisé illustré par les figures 2 et 3. Les données ont été stockées et moyennées pour en tirer un modèle prévisionnel de la consistance de cette formulation : la figure 7 montre l'évolution de la consistance en fonction du temps, en jours. La courbe montre que la crème a atteint son degré de maturité au bout d'environ 120 jours et que la consistance ne varie pas notablement jusqu'à environ 600 jours. Elle permet d'en déduire un modèle prévisionnel de commercialisation. Le stockage et la vente doivent être organisés de telle façon que le consommateur reçoive son produit environ 4 mois après la date de fabrication. A partir de ce moment, sa consistance ne se modifiera pas notablement pendant 13 mois environ.

### Exemple 5

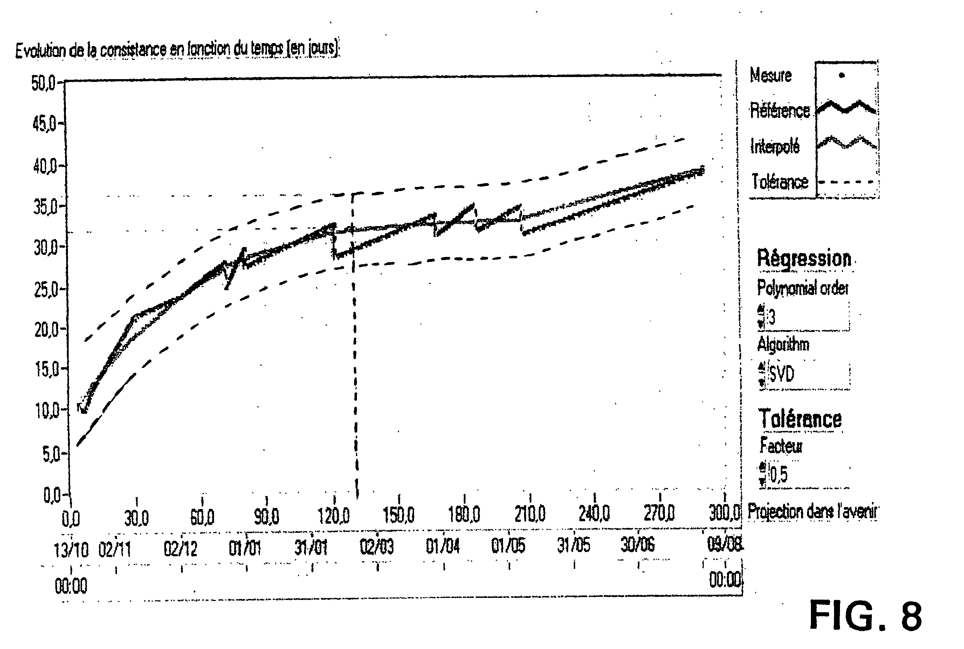

L'évolution de la consistance en fonction du temps de trois lots d'une même formulation d'une crème cosmétique est mesurée et enregistrée à l'aide du dispositif informatisé illustré par les figures 2 et 3 dans les mêmes conditions opératoires que dans l'exemple 4. Les données stockées sont présentées sur la figure 8; les deux courbes en trait plein représentent respectivement une simple interpolation linéaire des mesures et une courbe lissée obtenue par régression polynomiale. La consistance atteint après 130 jours une valeur acceptable sous l'aspect de la "psycho-acceptance" et qui reste à peu près constante. L'intervalle de tolérance sur les valeurs de la consistance est représentée par les deux courbes en traits pointillés : on constate que si une mesure est faite sur un échantillon sept jours après la fabrication et que la valeur de la consistance mesurée à ce moment est très proche de la courbe interpolée (valeur d'environ 10 à sept jours), la maturation ultérieure est suffisamment proche de l'évolution moyenne pour que la consistance à 130 jours se situe à l'intérieur de l'intervalle de tolérance.

Le procédé de mesure et le dispositif selon l'invention offrent une possibilité pratique et fiable d'effectuer des mesures de consistance aussi bien comparatives de divers produits à une même époque, que comparatives du même produit à diverses époques, en offrant la garantie que l'appareil de mesure, à paramètres opératoires constants, n'induit pas d'erreur non décelable, cela en raison de son agencement et de la faculté d'étalonner toujours de façon exacte cet appareil.

Pour une sonde de dimensions données, une course et une vitesse de descente données, deux appareils selon l'invention employant des composants différents (moteur, support mobile de sonde, capteur de force) donneront la même mesure, pourvu que l'abaissement du plateau pendant la descente de la sonde reste négligeable par rapport à sa course.

## Revendications

1. Procédé de mesure pénétrométrique de la consistance d'une substance, dans lequel on place un échantillon (7) de ladite substance sur un support mobile (8, 18) constituant un plateau pour l'échantillon, un capteur de force (28) supportant ledit plateau, on fait pénétrer du haut vers le bas une sonde calibrée (6, 16) dans cet échantillon, selon une course à accomplir dans la masse de l'échantillon et selon une vitesse préalablement choisie, et on mesure la force verticale dirigée vers le bas appliquée audit support à au moins un moment prédéterminé de la course de la sonde, **caractérisé en ce que** dans un premier temps on amène la sonde au contact de l'échantillon et, à cet instant, on recueille une valeur zéro de ladite force et une valeur zéro de la course, puis on donne à la sonde ladite vitesse préalablement choisie, on commande l'exécution de ladite course à ladite vitesse et on recueille à chacun desdits au moins un moment prédéterminé la valeur des la course et, simultanément, la valeur de la force, ledit capteur de force (28) étant choisi de sorte que ledit déplacement vertical du support est négligeable par rapport à la course de la sonde.

2. Procédé de mesure selon la revendication 1, **caractérisé en ce que** l'on recueille la valeur de ladite force au moment où la sonde (6, 16) a parcouru l'entier de ladite course.

3. Procédé de mesure selon la revendication 1, **caractérisé en ce que** l'on enregistre la variation de ladite force pendant la totalité du déplacement de la sonde (6, 16) dans ledit échantillon (7).

4. Procédé de mesure selon l'une des revendications 1 à 3, **caractérisé en ce que** la substance est conservée dans un récipient préalablement à la mesure, qu'elle est présentée dans ledit récipient à la sonde, sans avoir subi de contrainte mécanique avant ladite mesure.

5. Dispositif pour la mise en oeuvre d'un procédé selon l'une des revendications précédentes, comportant un moteur (4, 14) imprimant un mouvement vertical à une sonde (6, 16), un dispositif électronique de commande du moteur comprenant des moyens pour prédéterminer la course de la sonde et la vitesse de son mouvement de pénétration dans un échantillon (7), un support mobile (8, 18) constituant un plateau sur lequel l'échantillon peut être placé de manière que le mouvement de la sonde amène celle-ci au contact de l'échantillon, un capteur de force (28) supportant ledit plateau, **caractérisé en ce que** le capteur de force (28) est choisi de sorte que l'amplitude du déplacement vertical du plateau est négligeable par rapport à la course de la sonde, et **en ce que** ledit dispositif de mise en oeuvre comporte des moyens (4, 21, 22) pour amener la sonde (6) au contact de l'échantillon (7) et, à cet instant, recueillir des valeurs de zéro de la force et de la course, puis imposer au moteur (4, 14) une valeur prédéterminée et constante de la vitesse et une valeur prédéterminée de la course à effectuer, et recueillir à au moins un moment de la course des valeurs simultanées de la course effectuée et de la force mesurée.

6. Dispositif selon la revendication 5, **caractérisé en ce que** ledit capteur de force (28) est une cellule de charge comprenant un dispositif de guidage (29, 30, 31) assurant un soutien élastique à un enregistreur de charge, ledit dispositif de guidage constituant le seul support dudit plateau (8, 18).

7. Dispositif selon l'une des revendications 5 à 6, **caractérisé en ce que** le moteur est un moteur pas à pas, à courant continu ou un moteur linéaire.

8. Dispositif selon la revendication 7, **caractérisé en ce que** le dispositif de commande comporte un moyen de comptage d'impulsions et **en ce que** la course de la sonde est déterminée par le comptage desdites impulsions.

9. Dispositif selon la revendication 8, **caractérisé en ce qu'**il comporte une horloge délivrant un signal de fréquence au dispositif de commande pour déterminer la fréquence des impulsions délivrées au moteur et la vitesse de la sonde et un dispositif d'acquisition du signal délivré par ledit capteur de force (28).

10. Dispositif selon la revendication 9, **caractérisé en ce que** le dispositif de commande comporte des moyens pour fournir au dispositif d'acquisition du signal une commande de mise à zéro lorsque l'échantillon est placé sur le plateau, avant que la sonde ne soit en contact avec l'échantillon, et pour fournir à un organe de comptage des impulsions une commande de mise à zéro au moment où le contact entre la sonde et l'échantillon est révélé par la première valeur non-nulle du signal après mise à zéro, de sorte que le comptage des impulsions, qui détermine la course de la sonde, commence à ce moment.

11. Dispositif selon l'une des revendications 5 à 10, **caractérisé en ce qu'**il comporte un organe de mesure de la température de l'échantillon, en particulier **en ce que** l'organe de mesure de la température comporte une thermistance (25) placée à même la sonde (6).

12. Dispositif selon l'une des revendications 5 à 7, **caractérisé en ce que** le moteur et le capteur de force sont reliés à un ordinateur agencé pour gérer par un logiciel spécifique tout ou partie des fonctions de commande du moteur.

13. Dispositif selon la revendication 12, **caractérisé en ce que** ledit logiciel spécifique gère le comptage des impulsions délivrées au moteur, la délivrance d'un signal de fréquence, la mise à zéro du signal lorsque l'échantillon est placé sur le plateau, ainsi que la mise à zéro du compteur d'impulsions au moment où le contact entre la sonde et l'échantillon est révélé par la première valeur non-nulle du signal, et la collecte de la valeur donnée par le capteur de force lorsque le compteur d'impulsion atteint un nombre prédéterminé et/ou la collecte de toutes les valeurs données par ledit capteur à partir dudit moment où le contact entre la sonde et l'échantillon est révélé.

14. Dispositif selon les revendications 11 et 13, **caractérisé en ce qu'**il est conformé pour que ledit logiciel spécifique gère la collecte de la valeur de la température.

## Claims

1. Method of penetrometric measurement of the consistency of a substance, in which a sample (7) of said substance is placed on a mobile support (8, 18), constituting a platform for the sample, a force sensor (28) supporting said platform, in which a calibrated probe (6, 16) is inserted in this sample from the top down, following a travel to be completed in the mass of the sample and according to a previously selected speed, and in which the downward vertical force applied to said support is measured at least at one pre-determined moment of the travel of the probe, **characterised in that** the probe is initially placed in contact with the sample and, at this time, a zero value of said force and a zero value of the travel are registered, then the previously selected speed is transmitted to the probe, the execution of said travel at said speed is controlled and the value of the travel and, simultaneously, the value of the force are registered at each of said at least one pre-determined moments, said force sensor (28) being selected such that said vertical movement of the support is negligible in relation to the travel of the probe.

2. Measurement method according to claim 1, **characterised in that** the value of said force is registered at the moment when the probe (6, 16) has reached the end of said travel.

3. Measurement method according to claim 1, **characterised in that** the variation of said force is registered throughout the entire movement of the probe (6, 16) in said sample (7).

4. Measurement method according to one of claims 1 to 3, **characterised in that** the substance is kept in a container prior to the measurement, **in that** it is presented in said container to the probe, without having been subjected to any mechanical constraints prior to said measurement.

5. Device for the implementation of a method according to one of the preceding claims, comprising a motor (4, 14) transmitting a vertical movement to a probe (6, 16), an electronic device for controlling the motor comprising means for pre-determining the travel of the probe and the speed of its penetration movement in a sample (7), a mobile support (8, 18) which forms a platform on which the sample can be placed so that the movement of the probe brings the latter into contact with the sample, a force sensor (28) supporting said platform, **characterised in that** the force sensor (28) is selected so that the range of vertical movement of the platform is negligible in relation to the travel of the probe, and **in that** said device for implementation comprises means (4, 21, 22) for bringing the probe (6) into contact with the sample (7) and, at such time, for registering the zero values of the force and the travel, and then for transmitting to the motor (4, 14) a predetermined, constant speed value and a predetermined value of the travel to be completed, and for registering at least at one moment of the travel, the simultaneous values of the travel made and the force measured.

6. Device according to claim 5, **characterised in that** said force sensor (28) is a load cell comprising a guide device (29, 30, 31) providing an elastic support for a load recorder, said guide device constituting the sole support of said platform (8, 18).

7. Device according to one of the claims from 5 to 6, **characterised in that** the motor is a stepping motor, a direct-current motor or a linear motor.

8. Device according to claim 7, **characterised in that** the control device comprises means for counting impulses and **in that** the travel of the probe is determined by the counting of said impulses.

9. Device according to claim 8, **characterised in that** it comprises a clock that transmits a frequency signal to the control device in order to determine the frequency of the impulses transmitted to the motor and the speed of the probe and a device for acquiring the signal transmitted by said force sensor (28) .

10. Device according to claim 9, **characterised in that** the control device comprises means for supplying the signal-acquisition device with a command to reset it to zero when the sample is placed on the platform, before the probe is in contact with the sample, and to supply an impulse-counting element with a command to reset it to zero at the time when the contact between the probe and the sample is revealed by the first non-zero value of the signal after resetting to zero, so that the counting of the impulses, which determines the travel of the probe, starts at that moment.

11. Device according to one of claims 5 to 10, **characterised in that** it comprises an element for measuring the temperature of the sample, in particular **in that** the temperature-measuring element comprises a thermistor (25) placed in the actual probe (6).

12. Device according to one of the claims from 5 to 7, **characterised in that** the motor and the force sensor are connected to a computer set up to manage, by means of a specific software application, all or part of the motor control functions.

13. Device according to claim 12, **characterised in that** said specific software application manages the counting of the impulses transmitted to the motor, the transmission of a frequency signal, the resetting to zero of the signal when the sample is placed on the platform, as well as the resetting to zero of the impulse counter at the time when the contact between the probe and the sample is revealed by the first non-zero value of the signal, and the recording of the value provided by the force sensor when the impulse counter reaches a pre-determined number and/or the recording of all the values provided by said sensor as of said moment when the contact between the probe and the sample is revealed.

14. Device according to claims 11 and 13, **characterised in that** it is designed so that said specific software application manages the recording of the temperature value.

## Patentansprüche

1. Verfahren zur penetrometrischen Messung der Konsistenz einer Substanz, bei dem eine Probe (7) dieser Substanz auf eine bewegliche Stützeinrichtung (8, 18) aufgesetzt wird, die eine Platte für die Probe ausbildet, wobei ein Kraftsensor (28) diese Platte abstützt, bei dem man eine kalibrierte Sonde (6, 16) von oben nach unten in die Probe längs eines in der Masse der Probe zu hinterlegenden Weges mit einer vorab gewählten Geschwindigkeit eindringen lässt, und bei dem die vertikale, nach unten gerichtete und auf die Stützeinrichtung ausgeübte Kraft in zumindest einem vorbestimmten Moment längs des Weges der Sonde gemessen wird, **dadurch gekennzeichnet, dass** in einem ersten Zeitintervall die Sonde in Kontakt mit der Probe gebracht wird und in diesem Moment ein Wert Null für die Kraft und ein Wert Null für den Weg gesetzt wird, dass anschließend die Sonde auf die vorab gewählte Geschwindigkeit gebracht wird, dass die Ausführung des Weges mit dieser Geschwindigkeit eingeleitet wird und zu jedem des zumindest eines vorbestimmten Momentes der Wert des Weges und gleichzeitig der Wert der Kraft aufgenommen wird, wobei der Kraftsensor (28) so gewählt ist, dass die vertikale Verschiebung der Stützeinrichtung gegenüber dem Weg der Sonde vernachlässigbar ist.

2. Messverfahren nach Anspruch 1, **dadurch gekennzeichnet, dass** der Wert der Kraft in dem Moment aufgenommen wird, an dem die Sonde (6, 16) den gesamten Weg durchlaufen hat.

3. Messverfahren nach Anspruch 1, **dadurch gekennzeichnet, dass** die Änderung der Kraft während des gesamten Weges, den die Sonde (6, 16) in der Probe zurücklegt, aufgenommen wird.

4. Messverfahren nach einem der Ansprüche 1 bis 3, **dadurch gekennzeichnet, dass** die Substanz in einem Behälter vor der Messung so aufgenommen ist, dass sie in diesem Behälter der Sonde zugewandt ist, und ohne dass sie vor der Messung mechanischen Belastungen ausgesetzt wurde.

5. Vorrichtung zur Durchführung eines Verfahrens nach einem der vorhergehenden Ansprüche, mit einem Motor (4, 14), der eine Sonde (6, 16) vertikal bewegt, mit einer elektronischen Steuereinheit für den Motor, die Mittel zum Vorbestimmen des Weges der Sonde und deren Eindringgeschwindigkeit in eine Probe (7) umfasst, mit einer beweglichen Stützeinrichtung (8, 18), die eine Platte ausbildet, auf der die Probe so aufgesetzt werden kann, dass die Bewegung der Sonde diese in Kontakt mit der Probe bringt, wobei ein Kraftsensor (28) diese Platte abstützt, **dadurch gekennzeichnet, dass** der Kraftsensor (28) so gewählt ist, dass die Amplitude der vertikalen Verschiebung der Platte im Vergleich zum Weg der Sonde vernachlässigbar ist, und dass die Vorrichtung zur Durchführung des Verfahrens Einrichtungen (4, 21, 22) umfasst, um die Sonde (6) in Kontakt mit der Probe (7) zu bringen und in diesem Moment für die Kraft und den Weg jeweils die Werte Null zu setzen, anschließend dem Motor (4, 14) einen vorbestimmten konstanten Wert für die Geschwindigkeit und einen vorbestimmten Wert für den auszuführenden Weg aufzuerlegen und an zumindest einem Zeitpunkt des Weges gleichzeitig Werte für den zurückgelegten Weg und die gemessene Kraft aufzunehmen.

6. Vorrichtung nach Anspruch 5, **dadurch gekennzeichnet, dass** der Kraftsensor (28) eine Belastungszelle ist, die eine Führungseinrichtung (29, 30, 31) umfasst, die eine elastische Halterung für eine Lastaufzeichnungseinrichtung gewährleistet, wobei die Führungseinrichtung die einzige Stützeinrichtung für die Platte (8, 18) bildet.

7. Vorrichtung nach einem der Ansprüche 5 und 6, **dadurch gekennzeichnet, dass** der Motor ein Schrittmotor, ein Gleichstrommotor oder ein Linearmotor ist.

8. Vorrichtung nach Anspruch 7, **dadurch gekennzeichnet, dass** die Steuereinrichtung eine Einrichtung zum Zählen von Impulsen umfasst, und dass der Weg der Sonde durch das Zählen dieser Impulse bestimmt wird.

9. Vorrichtung nach Anspruch 8, **dadurch gekennzeichnet, dass** sie einen Taktgeber, der ein Frequenzsignal an die Steuereinrichtung liefert, um die Frequenz der an den Motor gelieferten Impulse und die Geschwindigkeit der Sonde zu bestimmen, und eine Einrichtung zum Erfassen des durch den Kraftsensor (28) gelieferten Signales umfasst.

10. Vorrichtung nach Anspruch 9, **dadurch gekennzeichnet, dass** die Steuereinrichtung Mittel zum Liefern eines Befehlssignales zum Nullsetzen an die Einrichtung zum Erfassen des Signales umfasst, wenn die Probe auf der Platte aufgesetzt ist und die Sonde gerade in Kontakt mit der Probe ist, und um ein Befehlssignal zum Nullsetzen an eine Impulszählvorrichtung in dem Moment zu liefern, in dem der Kontakt zwischen der Sonde und der Probe durch einen ersten Nicht-Nullwert des Signales nach dem Nullsetzen festgestellt wird, derart, dass die Zählung der Impulse, die den Weg der Sonde bestimmen, in diesem Moment beginnt.

11. Vorrichtung nach einem der Ansprüche 5 bis 10, **dadurch gekennzeichnet, dass** sie eine Messeinrichtung für die Temperatur der Probe umfasst, insbesondere dass die Messeinrichtung für die Temperatur einen Termistor (25) umfasst, der direkt an der Sonde (6) angebracht ist.

12. Vorrichtung nach einem der Ansprüche 5 bis 7, **dadurch gekennzeichnet, dass** der Motor und der Kraftsensor mit einem Rechner verbunden sind, der eingerichtet ist, um mit Hilfe einer spezifischen Software alle oder einen Teil der Funktionen zum Steuern des Motors auszuführen.

13. Vorrichtung nach Anspruch 12, **dadurch gekennzeichnet, dass** die spezielle Software die Zählung von an den Motor gelieferten Impulsen, die Abgabe eines Frequenzsignales, das Nullsetzen des Signales, im Moment wo die Probe auf der Platte abgesetzt ist, ebenso das Nullsetzen des Impulszählers im Moment, in dem der Kontakt zwischen der Sonde und der Probe durch den ersten Nicht-Nullwert des Signales festgestellt wird, und das Aufnehmen des durch den Kraftsensor abgegebenen Wertes, während der Impulszähler eine vorbestimmte Anzahl erreicht, und/oder das Aufnehmen aller Werte, die durch den Kraftaufnehmer ab dem Moment, in dem der Kontakt zwischen der Sonde und der Probe festgestellt ist, abgegeben werden, verwaltet.

14. Vorrichtung nach den Ansprüchen 11 und 13, **dadurch gekennzeichnet, dass** sie dergestalt ausgebildet ist, dass die spezifische Software das Aufnehmen des Temperaturwertes ausführt.
